# EUROPÄISCHE PATENTSCHRIFT

(11) **EP 2 799 001 B1**
(45) Veröffentlichungstag und Bekanntmachung des Hinweises auf die Patenterteilung: **02.12.2015**
(21) Anmeldenummer: 14165719.7
(22) Anmeldetag: 24.04.2014
(51) Int. Cl.: A61B 1/005, A61M 25/01, A61B 17/00, A61B 17/29

(54) **Medizinisches Instrument mit arretierbarer Abwinkelsteuerung**
Medical instrument with adjustable angle control
Instrument médical doté d'une commande de pliage pouvant être bloquée

(30) Priorität: 02.05.2013 US 201313886006
(43) Veröffentlichungstag der Anmeldung: 05.11.2014
(73) Patentinhaber: Karl Storz GmbH & Co. KG, 78532 Tuttlingen (DE)
(72) Erfinder: Bacher, Uwe, 78532 Tuttlingen (DE); Deufel, Egon, 78567 Fridingen (DE); Zahler, Sabine, 85591 Vaterstetten (DE)
(74) Vertreter: Witte, Weller & Partner Patentanwälte mbB

(56) Entgegenhaltungen:
- EP-A1- 0 306 723
- EP-A1- 2 027 820
- EP-A1- 2 106 751
- WO-A2-2007/081706
- WO-A2-2008/045374
- US-A- 5 766 196
- US-A- 5 976 075
- US-A1- 2002 165 484
- US-A1- 2004 193 016

## Beschreibung

Die Erfindung betrifft ein medizinisches Instrument, mit einem Schaft, dessen distales Ende abwinkelbar ist, mit einer am proximalen Ende des Schaftes angeordneten Handhabe, mit einem Abwinkelmechanismus zum Abwinkeln des Schaftes, wobei der Abwinkelmechanismus ein um eine Schwenkachse verschwenkbares Steuerelement aufweist, das an einer Außenseite der Handhabe angeordnet ist, und dessen Verschwenken um die Schwenkachse ein Abwinkeln des Schaftes verursacht, und mit einem Arretiermechanismus zum Arretieren des verschwenkbaren Steuerelementes in unterschiedlichen Schwenkstellungen, wobei der Arretiermechanismus ein Betätigungselement aufweist, dessen Betätigen den Arretiermechanismus löst.

Ein derartiges medizinisches Instrument ist aus der EP 2 106 751 A1 bekannt.

Medizinische Instrumente mit einem abwinkelbaren Ende des Schaftes erlauben dem Operateur wesentlich mehr Handhabungsfreiheitsgrade im Bereich des distalen Endes des Instrumentes.

Bei flexiblen Schäften besteht die Möglichkeit, die Schäfte über Körperkanäle, beispielsweise die Bronchien, die Speiseröhre oder den Darm in den Körper einzuführen. Durch das Abwinkeln des distalen Endes des Schaftes können die zuvor erwähnten zusätzlichen Freiheitsgrade der Handhabung durchgeführt werden.

Die Handhabungen sind vielerlei, so können es Fass- oder Präpariervorgänge, reine Beobachtungsvorgänge oder auch diese Vorgänge kombiniert sein.

Zur Steuerung der Abwinklung des Schaftes ist ein Abwinkelmechanismus vorhanden, der Steuerseile aufweist, die im abwinkelbaren Bereich des Schaftes an Stellen abseits der Mittellängsachse des Schaftes befestigt sind. Durch Ziehen an einem Steuerseil und Nachschieben des anderen Steuerseiles kann die Krümmung oder Abwicklung des Schaftes ausgelöst werden. Proximalseitig können die Steuerseile auf einem scheiben- oder trommelartigen Körper aufgebracht und dort fixiert werden. Ein Drehen dieses Körpers bewirkt ein Ziehen an einem Steuerseil und ein Schieben am anderen Steuerseil. Es können auch verschwenkbare Zahnräder vorgesehen sein, die in eine Verzahnung am Ende der Steuerseile eingreifen. Zur Steuerung dieser Bewegungen ist an der Außenseite der Handhabe ein verschwenkbares Steuerelement vorgesehen, das, meist über einen äußeren Hebel, mit der Drehachse des drehbaren Körpers verbunden ist, um die sich die im Innern der Handhabe angeordneten Mechanismusteile drehen, also beispielsweise eine Trommel, an der die Steuerseile befestigt sind. Bei der Durchführung der unterschiedlichen Manipulationen ist es erwünscht, dass der abgewinkelte Schaft in bestimmten abgewinkelten Stellungen verbleibt. Dazu ist ein Arretiermechanismus vorgesehen. Der Arretiermechanismus weist ein Betätigungselement auf, dessen Betätigung den Arretiermechanismus löst und dadurch dann die Verschwenkung des verschwenkbaren Steuerelementes freigibt.

Üblicherweise ist der Arretiermechanismus so ausgebildet, dass, falls das Betätigungselement nicht bewegt wird, der Arretiermechanismus automatisch arretiert, also die Bewegung des verschwenkbaren Steuerelementes sperrt.

Bei der eingangs erwähnten EP 2 106 751 A1 ist das Betätigungselement am verschwenkbaren Steuerelement angeordnet. Dies erlaubt ein sehr ergonomisches Handhaben des Instrumentes. Die Handhabungsperson kann beispielsweise das Betätigungselement, das als eine Art Drücker ausgebildet ist, eindrücken, um dadurch den Arretiermechanismus zu lösen. Dazu wird ein Finger einer Hand auf das Betätigungselement gelegt. Nach Lösen des Arretiermechanismus kann dann mit demselben Finger das verschwenkbare Steuerelement bewegt werden.

Ein ähnliches Instrument ist aus der US 2002/0165484 A1 bekannt, bei der das Steuerelement zum Lösen der Arretierung gegen die Kraft einer Feder bewegt und danach verschwenkt werden kann.

Es ist Aufgabe der vorliegenden Erfindung ein medizinisches Instrument der eingangs genannten Art dahingehend weiterzuentwickeln, dass es zum einen ergonomisch und einfach handhabbar ist und zum andern einfach aufgebaut ist und einer robusten Handhabung im praktischen Einsatz widerstehen kann.

Erfindungsgemäß wird die Aufgabe dadurch gelöst, dass von der Außenseite der Handhabe eine Platte hochsteht, an deren gegenüberliegenden Seitenwänden Bremselemente anliegen, die mit dem verschwenkbaren Steuerelement verbunden sind und die derart in Richtung der Seitenwände vorgespannt sind, dass diese ein Verschwenken des Steuerelementes arretieren, und dass das Betätigungselement derart mit den Bremselementen verbunden ist, dass ein Betätigen des Betätigungselementes die Bremselemente aus dem arretierenden Eingriff mit der Platte bringen.

Diese Maßnahmen haben den Vorteil, dass eine einfache und sichere Handhabung dadurch ermöglicht und gewährleistet ist, dass durch die Betätigung des Betätigungselements die Bremselemente aus dem arretierenden Eingriff mit der Platte gebracht werden, und zwar soweit, dass das Steuerelement zum Abwinkeln des Schaftes verschwenkt werden kann. Eine robuste und einfache Bauweise ist dadurch möglich, dass die von der Außenseite der Handhabe abstehende Platte einfach und stabil aufbaubar ist. Diese kann bereits bei der Herstellung der Handhabe in diese integriert bzw. eingebaut werden. Die Platte wirkt quasi wie eine Art Bremsscheibe. Am verschwenkbaren Steuerelement sind Bremselemente vorgesehen, die in arretierendem Eingriff mit den gegenüberliegenden Seitenwänden der Platte treten. Da die Bremselemente in Richtung auf die Seitenwände vorgespannt sind, arretieren diese das verschwenkbare Steuerelement gegenüber der Handhabe automatisch. Erst durch Betätigen des Betätigungselementes wird dieser arretierende Eingriff zumindest soweit aufgehoben, dass ein Bewegen des verschwenkbaren Steuerelementes längs der Platte möglich ist. Dazu ist es nicht notwendig, dass die Bremselemente vollständig von den Seitenwänden abheben, es kann auch ausreichen, dass nur der Anpressdruck verringert wird. Das gibt der Handhabungsperson ein sicheres Steuergefühl beim Verschwenken des Schaftes. So können beispielsweise kleine Korrekturen der Verschwenkstellung noch dadurch bewerkstelligt werden, dass der Anpressdruck durch das Betätigungselement gerade soweit verringert wird, dass eine Verschwenkung möglich ist, jedoch nicht eine vollständig reibungs- oder bremseingrifflose Bewegung. Nach Freigabe des Betätigungselementes liegen dann die Bremselemente wieder unter dem vollen Anpressdruck an den Seitenwänden der Platte an.

Der jeweils geeignete Anpressdruck kann durch die entsprechende Vorspannung eingestellt werden. Ist das medizinische Instrument vorwiegend ein Beobachtungsinstrument oder ein Instrument, bei dessen Handhabung keine allzu starken Kräfte auf den abgewinkelten Schaft dahingehend einwirken, dass dieser aus der gekrümmten Stellung wieder zurückgestellt werden soll, reichen schon relativ geringe Anpresskräfte aus.

Sollen Manipulationen durchgeführt werden, bei denen insbesondere auf das distale Ende seitlich relativ starke Kräfte einwirken, wie beispielsweise bei Nähvorgängen oder Koagulationsvorgängen, müssen dann die Anpresskräfte entsprechend hoch eingestellt werden.

Die Montage ist ebenfalls einfach durchzuführen, es muss lediglich das verschwenkbare Steuerelement sowie das Betätigungselement so an die Außenseite der Handhabe herangebracht und montiert werden, dass diese beiden Elemente einerseits mit der Platte und andererseits mit den mit der Schwenkachse verbundenen Arm oder Hebel des Abwinklungsmechanismus zusammenwirken können. Neben einer einfachen Montage ist auch ein einfaches Zerlegen beispielsweise für Reinigungsvorgänge möglich. Dazu wird die Verbindung des Arms oder Hebels und der Schwenkachse gelöst und der Zusammenbau aus Betätigungselement und Steuerelement kann von der hochstehenden Platte abgehoben werden.

In einer weiteren Ausgestaltung der Erfindung ist das verschwenkbare Steuerelement samt dessen Bremselementen längs der Platte bewegbar.

Diese Maßnahme hat den Vorteil, dass in allen möglichen Verschwenkstellungen des Steuerelementes ein Eingriff der Bremselemente mit der Platte möglich ist.

In einer weiteren Ausgestaltung der Erfindung ist das Steuerelement über einen Arm mit der Schwenkachse verbunden, und eine radial äußere Kante der Platte weist eine kreisförmige Krümmung auf, deren Krümmungsradius der Schwenkachse entspringt.

Diese Maßnahme hat den Vorteil, dass die Platte die Form eines Kreisabschnittes aufweist, der von der Außenseite der Handhabe vorsteht, wobei dessen äußere Kante auf einem Kreis liegt, dessen Zentrum in der Schwenkachse liegt. Dadurch kann das Steuerelement, das ja um diese Schwenkachse verschwenkt wird, besonders sicher längs der Platte verfahren werden. Zugleich erlaubt diese Geometrie dieses Kreis- oder Plattensegment wenig raumergreifend zu bauen, jedoch ausreichend stabil, um sicherzustellen, dass in allen Verschwenkstellungen des Steuerelementes die Bremselemente mit den Seitenwänden der Platte in einen Brems- bzw. Arretierkräfte übertragenden Eingriff treten können.

In einer weiteren Ausgestaltung der Erfindung ist das Betätigungselement am äußeren Ende des Steuerelementes angeordnet und ist gegen die Kraft eines Federelementes auf das Steuerelement zu bewegbar, wodurch die Bremselemente von den Seitenwänden der Platte wegbewegbar sind.

Dies erlaubt eine besonders einfache und ergonomische Handhabung des Instrumentes. Die Handhabungsperson kann das Betätigungselement, das am äußeren Ende des Steuerelementes montiert ist, ergreifen, beispielsweise mit einem Finger einer Hand, die die Handhabe ergriffen hat und es in Richtung des Steuerelementes gegen die Kraft einer Feder bewegen. Bei dieser Bewegung werden die Bremselemente gelöst und der Zusammenbau aus Betätigungselement und Steuerelement kann nun zum Abwinkeln des Schaftes um die Schwenkachse verschwenkt werden. Wird das Betätigungselement freigegeben, wird das durch die Kraft der Feder wieder nach außen gedrückt und die Bremselemente legen sich an die Außenseite der Platte an.

In einer weiteren Ausgestaltung der Erfindung sind die Bremselemente jeweils als U-förmige Bügel ausgebildet, deren eines Ende mit dem Betätigungselement verbunden ist und deren anderes Ende an einer Seitenwand der Platte anliegt.

Diese Ausgestaltung als U-förmige Bügel erlaubt eine sehr kompakte Bauweise der Bremselemente, insbesondere in Zusammenhang mit der von außen nach innen gerichteten Steuerbewegung des Betätigungselementes.

In einer weiteren Ausgestaltung der Erfindung ist das eine Ende eines U-förmigen Bügels über einen Schwenkzapfen am Betätigungselement angebracht, so dass ein Bewegen des Betätigungselementes in Richtung der Platte ein Wegschwenken des anderen Endes von der jeweiligen Seitenwand der Platte bewirkt.

Durch diese Maßnahme kann auf konstruktiv einfache und sehr kompakt bauende Art und Weise die radial nach innen gerichtete lineare Eindrückbewegung des Betätigungselements in eine nach außen gerichtete abhebende Schwenkbewegung derjenigen Enden der Bügel umgesetzt werden, die in arretierendem Eingriff mit der Platte stehen. Diese kompakte Bauweise eröffnet die Möglichkeit, die Bremselemente im Innern des Steuerelementes anzuordnen, wobei diese durch das an der Außenseite des Steuerelementes angeordnete Betätigungselement bewegt werden.

Die erfindungsgemäße Ausgestaltung gibt der Handhabungsperson eine spürbare Rückmeldung, also ob die Bremselemente noch in voll sperrendem Eingriff mit der "Bremsscheibe" stehen, oder schon eine Verschwenkbewegung des Zusammenbaus aus Betätigungselement und Steuerelement, ggf. noch unter Überwindung eines "Bremswiderstandes" möglich ist.

In einer weiteren Ausgestaltung der Erfindung sind die Seitenwände der Platte aufgeraut.

Diese Maßnahme hat den Vorteil, dass der arretierende Eingriff zusätzlich verstärkt wird.

In einer weiteren Ausgestaltung der Erfindung sind die Bremselemente in dem Bereich, an denen diese mit den Seitenwänden der Platte in Berührung stehen, mit zumindest einem Bremskörper versehen.

Diese Maßnahme hat den Vorteil, dass die Bremskörper aus einem die Reibungskraft erhöhendem Material hergestellt werden können, quasi als Bremsklötze dienen. So können die Bremselemente selbst, beispielsweise, wenn sie als U-förmige Bügel ausgebildet sind, aus anderen Materialien hergestellt werden, in die die Bremskörper eingesetzt sind, um die Brems- bzw. Arretierwirkung zu erhöhen.

In einer weiteren Ausgestaltung der Erfindung sind zumindest in einer Seitenwand der Platte Vertiefungen vorgesehen, in die Vorsprünge der Bremselemente einrückbar sind.

Diese Maßnahme hat den Vorteil, dass neben der Arretierung durch reine Reibkraft bzw. Reibschluss auch zusätzlich noch ein Formschluss möglich ist, der eine besonders widerstandfähige Arretierung erlaubt.

Diese Ausgestaltung wird insbesondere dann vorgesehen sein, wenn bei der Handhabung des medizinischen Instrumentes starke Rückstellkräfte insbesondere im distalen Endbereich des gekrümmten Schaftes zu erwarten sind, die möglicherweise ein Verschieben der Bremselemente längs der Seitenwände verursachen könnten.

In einer weiteren Ausgestaltung der Erfindung ist eine Reihe an Vertiefungen vorgesehen, die längs einem Kreis angeordnet sind, dessen Radius der Schwenkachse entspringt.

Diese Maßnahme hat den Vorteil, dass zahlreiche mechanische Arretierstellen zur Verfügung gestellt werden, die längs einer gekrümmten Reihe angeordnet sind, die einer Kreislinie um die Schwenkachse entspricht.

Es versteht sich, dass die vorstehend genannten und die nachstehend noch zu erläuternden Merkmale nicht nur in den angegebenen Kombinationen sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: eine perspektivische Seitenansicht eines ersten Ausführungsbeispiels eines medizinischen Instrumentes im proximalen Endbereich des Schaftes mit der Handhabe,
- Fig. 2: einen teilweise gebrochenen Schnitt durch das Steuerelement längs des seitlichen Armes, über den dieses mit der Handhabe verbunden ist, wobei der arretierende Zustand dargestellt ist,
- Fig. 3: eine der Fig. 2 vergleichbare Darstellung, bei der die Bremselemente von der Platte abgehoben sind,
- Fig. 4: eine Seitenansicht des Endabschnittes von Fig. 1 von der gegenüberliegenden Seite her mit Erläuterungen zur Handhabung, und
- Fig. 5: eine der Darstellung von Fig. 4 entsprechende Seitenansicht eines weiteren Ausführungsbeispiels, bei der in der Platte in den Seitenwänden keine Vertiefungen vorhanden sind.

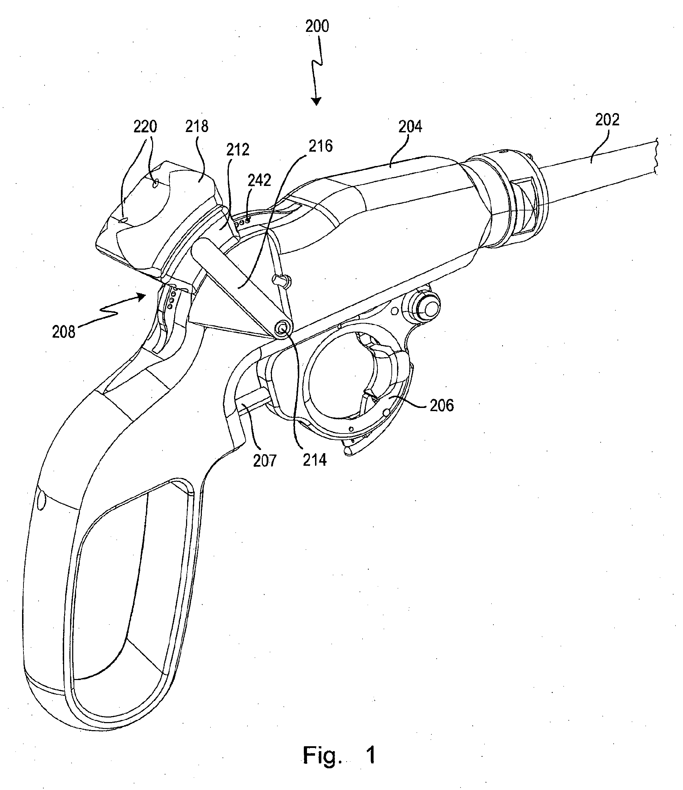

In den Fig. 1 bis 4 ist ein erstes Ausführungsbeispiel eines medizinischen Instrumentes dargestellt, das in seiner Gesamtheit mit der Bezugsziffer 200 versehen ist. Das medizinische Instrument 200 weist einen langerstreckten abwinkelbaren Schaft 202 auf, dessen proximales Ende mit einer Handhabe 204 versehen ist.

Das hier nicht dargestellte distale Ende des abwinkelbaren Schaftes 202 ist mit einem Werkzeug versehen, beispielsweise mit zwei Maulteilen zum Ergreifen eines Gewebestückes oder dergleichen, wie das bei solchen Instrumenten üblich ist.

An der Außenseite, in der Darstellung von Fig. 1 der Unterseite der Handhabe 204 ist ein bewegliches Griffteil 206 angeordnet, das längs einer Führungsstange 207 hin und her bewegbar ist.

Das bewegliche Griffteil 206 dient dazu, um das zuvor beschriebene Werkzeug am distalen Ende des Schaftes zu bewegen, beispielsweise ein bewegliches Maulteil gegenüber einem feststehenden Maulteil zu öffnen und zu schließen. Diese Bewegung wird durch ein Hin- und Herbewegen des beweglichen Griffteiles 206 längs der Führungsstange 207 gesteuert wie das aus dem medizinischen Instrumentenbau an sich bekannt ist.

Die Handhabe weist ferner ein pistolengriffartiges, hier nicht näher bezeichnetes, feststehendes Griffteil auf, über das die Handhabe 204 von einer Hand einer Bedienungsperson ergriffen werden kann. Zum Betätigen des beweglichen Griffteiles 206 weist dieses eine Fingeröse auf, in die ein Finger, beispielsweise der Zeigefinger einer Hand eingeschoben werden kann, die das medizinische Instrument 200 ergriffen hat.

Das medizinische Instrument 200 weist ferner einen Abwinkelmechanismus auf, der in seiner Gesamtheit mit der Bezugsziffer 208 bezeichnet ist. Der Abwinkelmechanismus 208 beinhaltet auch einen Arretiermechanismus, wie das nachfolgend noch erläutert wird.

Der Abwinkelmechanismus 208 weist ein verschwenkbares Steuerelement 212 auf, das um eine Schwenkachse 214 verschwenkbar ist. Das verschwenkbare Steuerelement 212 ist mit der Schwenkachse 214 über einen seitlich am Gehäuse der Handhabe 204 angeordneten Arm 216 verbunden. Der Arm 216 bewegt sich in einer seitlichen Aussparung im Gehäuse in der Handhabe 204 und ist in einem Schwenkbereich von etwa 45° um die Schwenkachse 214 verschwenkbar.

Die Schwenkachse 214 ist zugleich die Schwenkachse von Bauelementen im Innern der Handhabe 204, deren Verschwenkung das Abwinkeln des Schaftes 212 bewirken, wie das ebenfalls an sich bekannt ist. So kann beispielsweise die Schwenkachse 214 die Schwenkachse einer Trommel sein, an der zwei Enden von Steuerseilen befestigt sind, die durch den Schaft 202 zum distalen Ende führen. Beim Verschwenken der Trommel in der einen Richtung wird ein Seil in die Handhabe 204 eingezogen, das andere ausgeschoben, so dass dann das distale Ende des abwinkelbaren Schafts 202 in eine Richtung abgewinkelt wird, beispielsweise nach "oben". Beim Drehen in die entgegengesetzte Richtung wird das distale Ende in die entgegengesetzte Richtung abgewinkelt, beispielsweise nach "unten".

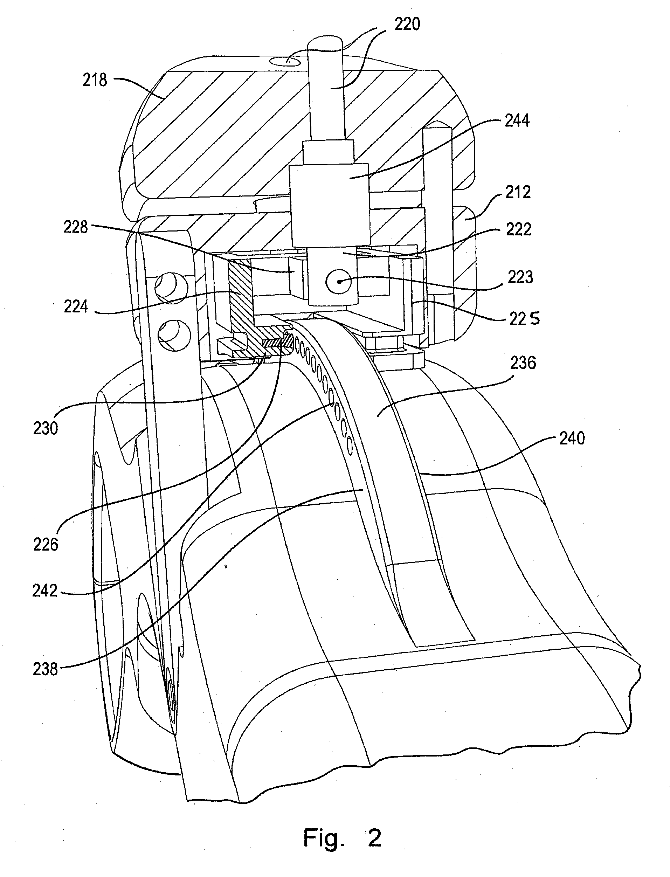

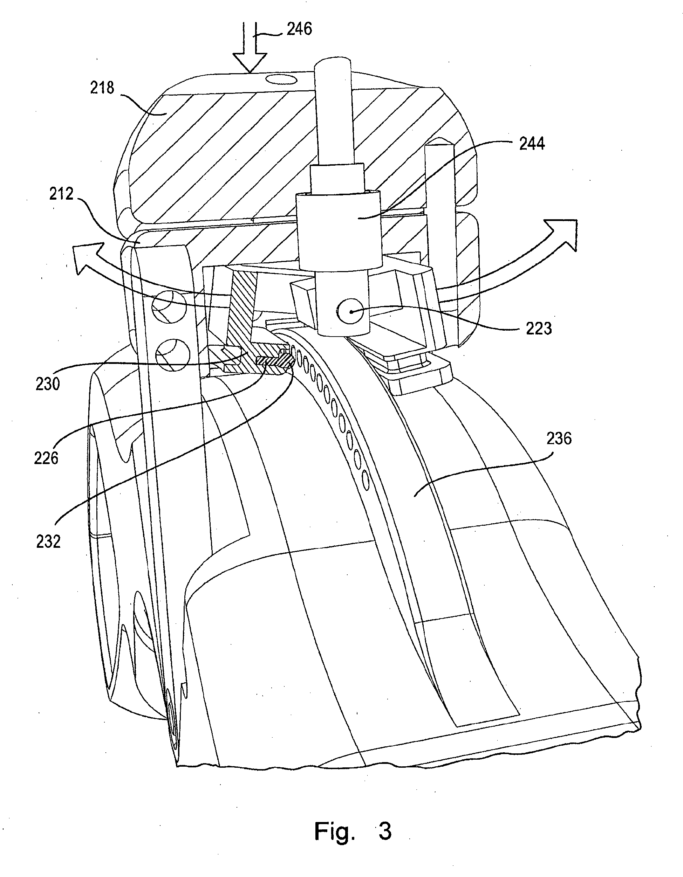

An der radial äußeren Seite des verschwenkbaren Steuerelementes 212 ist ein Betätigungselement 218 montiert, das Teil des Arretiermechanismus ist. Wie insbesondere aus den Schnittdarstellungen von Fig. 2 und 3 zu erkennen, ist das Betätigungselement 218 über zwei Steuerstangen 220 geführt am verschwenkbaren Steuerelement 212 aufgenommen. Zwischen den Steuerstangen 220 erstreckt sich eine Fingermulde, in die besonders ergonomisch die Fläche eines Daumens eingelegt werden kann.

Zumindest eine Steuerstange 220 ist an ihrem unteren Ende, in einem hier nicht näher bezeichneten Hohlraum im Steuerelement 212 mit einem darin aufgenommenen Bremselement 224 bzw. 225 verbunden.

Jedes der Bremselemente 224, 225 ist als ein U-förmiger Bügel ausgebildet.

Jedes der U-förmigen Bremselemente 224 bzw. 225 weist ein erstes äußeres Ende 228 auf, das über einen Schwenkzapfen 223 mit dem unteren Ende einer Steuerstange 220 verbunden ist. Die beiden ersten Enden 228 der beiden Bremselemente 224, 225 sind, in Längsrichtung des Zapfens 223 gesehen, hintereinander angeordnet und mit diesem verbunden.

Das zweite Ende 230 jedes Bremselementes 224, 225 ist an seinem äußeren Ende jeweils mit einem Bremskörper in Form eines Bremszapfens 226 versehen, dessen äußeres Ende 232, wie das insbesondere aus Fig. 3 ersichtlich ist, etwas von diesem zweiten Ende 230 vorsteht.

Von der oberen Außenseite der Handhabe 204 steht eine hochstehende Platte 236 vor, die die Form eines Kreisabschnittes aufweist. Die Platte 236 ist fest mit der Handhabe 204 verbunden.

Die Platte 236 weist eine radial äußere Kantenfläche auf, deren Krümmung einem Kreis entspricht, dessen Ursprung in der Schwenkachse 214 liegt. In den gegenüberliegenden äußeren Seitenwänden 238 und 240 der hochstehenden Platte 236 sind eine Reihe an Vertiefungen 242 angeordnet, wobei die Reihe ebenfalls auf einer Kreislinie liegt, deren Ursprung die Schwenkachse 214 ist. Die Form und Ausgestaltung der Vertiefungen 242 ist derart, dass in diese die Vorsprünge 232 der Bremszapfen 226 eintreten können, wie das beispielsweise aus Fig. 2 ersichtlich ist.

Die hochstehende Platte 236 und die Bremselemente 224, 225 in Zusammenhang mit den Steuerstangen 220 und dem Betätigungselement 218 bilden die wesentlichen Bauteile des Arretiermechanismus.

Um die Steuerstange 220 herum ist ein Federelement 244 angeordnet, das sich am unteren Ende auf dem Steuerelement 212 abstützt und am oberen Ende an dem Betätigungselement 218. Das Federelement 244 ist derart vorgespannt, dass es das Betätigungselement 218 nach außen gerichtet, weg vom Steuerelement 212 bewegt. Das Federelement 244 selbst kann aus einer Schraubenfeder, mehreren übereinander gelegten Tellerfedern oder auch als verformbar elastischer Kunststoffkörper ausgebildet sein.

In Fig. 2 ist eine Situation dargestellt, die der arretierenden Stellung entspricht. Die Kraft des Federelementes 244 hat das Betätigungselement 218 soweit nach außen gedrückt, dass die Bremselemente 224, 225 an den gegenüberliegenden Seitenwänden 238 und 240 der Platte 236 unter einem gewissen Anpressdruck anliegen.

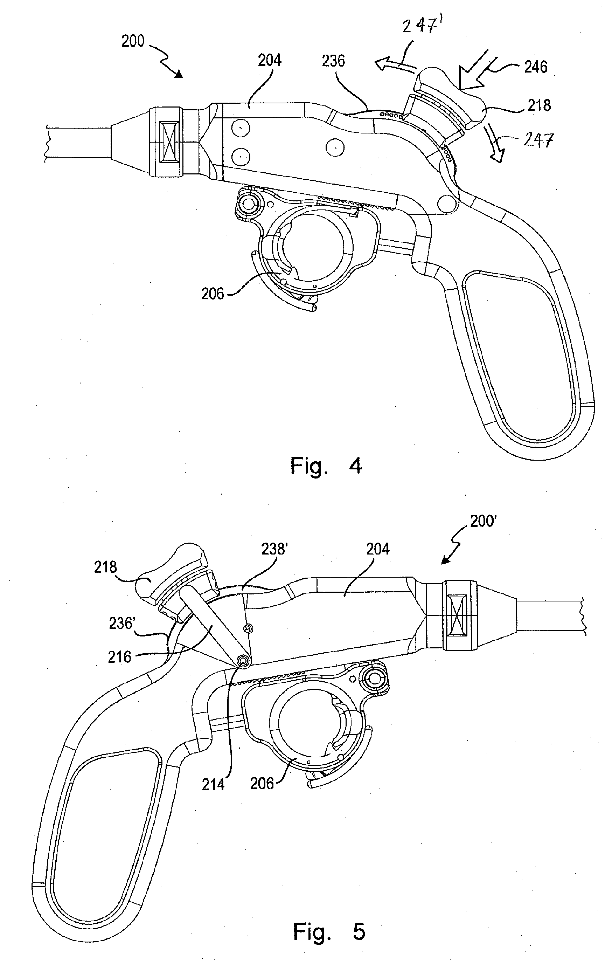

Wie insbesondere aus Fig. 2 zu erkennen, ist dabei ein Vorsprung 232 eines Bremszapfens 226 des Bremselementes 224 in eine entsprechende Vertiefung 242 in der Seitenwand 238 der Platte 236 eingerückt. Es kann ausreichend sein, nur auf dieser einen Seitenwand 238 solche Vertiefungen 242 vorzusehen. Es können solche auch auf der gegenüberliegenden Seitenwand vorgesehen sein, wie das insbesondere aus Fig. 4 ersichtlich ist.

Soll der Schaft 202 abgewinkelt werden, wird von außen auf das Betätigungselement 218 gedrückt, wie das in den Fig. 3 und 4 durch einen Pfeil 246 angedeutet ist. Dadurch bewegt sich auch der Schwenkzapfen 233 der Steuerstange 220 nach unten in den Innenraum des Steuerelementes 214 hinein, wodurch die beiden U-förmigen Bremselemente 214, 225 so nach außen verschwenkt werden, wie das in Fig. 3 durch die beiden Pfeile angedeutet ist. Bei dieser Verschwenkbewegung tritt beispielweise der Vorsprung 232 des Bremszapfens 226 aus der entsprechenden Vertiefung 242 in der Platte 236 aus. Nunmehr ist die Arretierung gelöst und das Steuerelement 212 kann längs der Platte 236, wie das in Fig. 4 durch die Pfeile 247 bzw. 247' dargestellt ist, bewegt werden. Bei dieser Schwenkbewegung wird das distale Ende des Schaftes 202 abgewinkelt.

Wird in einer bestimmten Verschwenkstellung des Steuerelementes 212 das Betätigungselement 218 freigegeben, drückt das Federelement 244 das Betätigungselement 218 wieder radial nach außen, so dass dann das zweite Ende 230 der Bremselemente 240, 250 wieder nach innen in Richtung der äußeren Seitenwände 238 und 240 der hochstehenden Platte 236 verschwenkt wird. Je nach Anordnung und Anzahl der Vertiefungen 242 greift dann der Vorsprung 232 eines Bremszapfens 226 wieder in eine solche Vertiefung 242 ein. Es können nur wenige solche Vertiefungen 242 vorgesehen sein und es können zwischen den einzelnen Vertiefungen 242 wesentliche größere Abstände bestehen. Die Anpresskraft mit der die zweiten Enden 230 an die Seitenwände 238, 240 durch das Federelement 244 gedrückt werden, ist so eingestellt, dass ein weiteres Verschwenken des Steuerelementes 212 gesperrt ist, auch wenn ein Bremszapfen 226 nicht in eine Vertiefung 242 eingerückt ist.

Es kann auch vorgesehen sein, solche Vertiefungen nur an bestimmten Stellen vorzusehen, die bestimmte Abwinkelstellungen des Schaftes entsprechen, beispielsweise 0°, 22,5° und 45° oder dergleichen.

In Fig. 5 ist ein zweites Ausführungsbeispiel eines medizinischen Instrumentes dargestellt, das in seiner Gesamtheit mit der Bezugsziffer 200' bezeichnet ist. Dieses weitere Ausführungsbeispiel ist im Prinzip gleich aufgebaut, wie das zuvor beschriebene Ausführungsbeispiel mit der Ausnahme, dass die Seitenwände 238' und 240' der hochstehenden Platte 236' keine Vertiefungen aufweisen. Ansonsten ist das Funktionsprinzip gleich, d.h. wenn das Betätigungselement 218 eingedrückt wird, lösen die Bremselemente und die Verschwenkung des Steuerelementes um die Schwenkachse 214 ist freigegeben.

In diesem Ausführungsbeispiel können die gesamten zweiten Enden 230 der Bremselemente mit einem Material mit einem hohen Reibungskoeffizienten versehen oder beschichtet sein, die quasi als Bremskörper fungieren. Zusätzlich können die Oberflächen der Seitenwände 238' und 240' aufgeraut sein, um den Reibungskoeffizienten zu erhöhen.

## Patentansprüche

1. Medizinisches Instrument, mit einem Schaft (202), dessen distales Ende abwinkelbar ist, mit einer am proximalen Ende des Schaftes (202) angeordneten Handhabe (204), mit einem Abwinkelmechanismus (208) zum Abwinkeln des Schaftes (202), wobei der Abwinkelmechanismus (208) ein um eine Schwenkachse (214) verschwenkbares Steuerelement (212) aufweist, das an einer Außenseite der Handhabe (204) angeordnet ist, und dessen Verschwenken um die Schwenkachse (214) ein Abwinkeln des Schaftes (202) verursacht, und mit einem Arretiermechanismus zum Arretieren des verschwenkbaren Steuerelementes (212) in unterschiedlichen Schwenkstellungen, wobei der Arretiermechanismus ein Betätigungselement (218) aufweist, dessen Betätigen den Arretiermechanismus löst, **dadurch gekennzeichnet, dass** von der Außenseite der Handhabe (204) eine Platte (236, 236') hochsteht, an deren gegenüberliegenden Seitenwänden (238, 238', 240) Bremselemente (224, 225) anliegen, die mit dem verschwenkbaren Steuerelement (212) verbunden sind und die derart in Richtung der Seitenwände (238, 238', 240) vorgespannt sind, dass diese eine Verschwenkung des verschwenkbaren Steuerelementes (212) arretieren, und dass das Betätigungselement (218) derart mit den Bremselementen (224, 225) verbunden ist, dass ein Betätigen des Betätigungselementes (218) die Bremselemente (224, 225) aus dem arretierenden Eingriff mit der Platte (236) bringen.

2. Medizinisches Instrument nach Anspruch 1, **dadurch gekennzeichnet, dass** das verschwenkbare Steuerelement (212) samt den Bremselementen (224, 225) längs der Platte (236, 236') bewegbar ist.

3. Medizinisches Instrument nach Anspruch 1 oder 2, **dadurch gekennzeichnet, dass** das Steuerelement (218) über einen Arm (216) mit der Schwenkachse (214) verbunden ist, und dass eine äußere Kante der Platte (236, 236') eine kreisförmige Krümmung aufweist, deren Krümmungsradius der Schwenkachse (214) entspringt.

4. Medizinisches Instrument nach einem der Ansprüche 1 bis 3, **dadurch gekennzeichnet, dass** das Betätigungselement (218) am äußeren Ende des verschwenkbaren Steuerelementes (214) angeordnet ist und gegen die Kraft eines Federelementes (244) auf das Steuerelement (212) zu bewegbar ist, wodurch die Bremselemente (224, 225) von den Seitenwänden (238, 238', 240) der Platte (236, 236') wegbewegbar sind.

5. Medizinisches Instrument nach einem der Ansprüche 1 bis 4, **dadurch gekennzeichnet, dass** die Bremselemente (224, 225) jeweils als U-förmige Bügel ausgebildet sind, deren eines Ende (228) mit dem Betätigungselement (218) verbunden ist, und deren anderes Ende (230) an einer Seitenwand (238, 238', 240) der Platte (236, 236') anlegbar ist.

6. Medizinisches Instrument nach Anspruch 5, **dadurch gekennzeichnet, dass** das eine Ende (228) des U-förmigen Bügels über einen Schwenkzapfen (223) am Betätigungselement (218) angebracht ist, so dass ein Bewegen des Betätigungselementes (218) in Richtung der Platte (236, 236') ein Wegschwenken des anderen Endes (230) des U-förmigen Bügels von der jeweiligen Seitenwand (238, 238', 240) der Platte (236, 236') bewirkt.

7. Medizinisches Instrument nach einem der Ansprüche 1 bis 6, **dadurch gekennzeichnet, dass** die Seitenwände (238, 238', 240) der Platte (236, 236') aufgeraut sind.

8. Medizinisches Instrument nach einem der Ansprüche 1 bis 7, **dadurch gekennzeichnet, dass** die Bremselemente (224, 225) in dem Bereich, an denen diese mit den Seitenwänden (238, 238', 240) der Platte (236, 236') in Berührung stehen, mit zumindest einem Bremskörper (226) versehen sind.

9. Medizinisches Instrument nach einem der Ansprüche 1 bis 8, **dadurch gekennzeichnet, dass** in einer Seitenwand (238, 238', 240) der Platte (236, 236') zumindest eine Vertiefung (242) vorgesehen ist, in die Vorsprünge (232) der Bremselemente (224, 225) einrückbar sind.

10. Medizinisches Instrument nach Anspruch 8 oder 9, **dadurch gekennzeichnet, dass** eine Reihe an Vertiefungen (242) vorgesehen ist, die längs einem Kreis angeordnet sind, dessen Radius der Schwenkachse (214) entspringt.

## Claims

1. Medical instrument, comprising a shaft (202) whose distal end in being bendable, with a handle (204) arranged at a proximal end of the shaft (202), a bend control mechanism (208) for bending the shaft (202), wherein the bend control mechanism (208) has a pivotable control element (212) which can be pivoted about a pivot axis (214) and which is arranged at an outer side of the handle (204), and a pivoting around the pivot axis (214) causes a bending of the shaft (202), and with a locking mechanism for locking the bendable control element (212) in different pivot positions, wherein the locking mechanism has a actuating element (218) whose actuating releases the locking mechanism, **characterized in that** a plate (236, 236') stands up from an outer surface of the handle (204), wherein brake elements (224, 225) rest against opposite side walls (238, 238', 240) thereof which brake elements are connected to the bendable control element (212) and are biased in such a way in direction to the side walls (238, 238', 240), that it lock a pivoting of the pivotable control element (212), and **in that** the actuating element (218) is connected to the brake elements (224, 225) in such a way, that an actuation of the actuating element (218) moves the brake elements (224, 225) out of the locking engagement with the plate (236).

2. Medical instrument of claim 1, **characterized in that** the pivotable control element (212) is movable together with the brake elements (224, 225) along the plate (236, 236').

3. Medical instrument of claims 1 or 2, **characterized in that** the control element (218) is connected to the pivot axis (214 via an arm (216), and **in that** the outer rim of the plate (236, 236') has a circular curvature, a radius of curvature origins from the pivot axis (214).

4. Medical instrument of anyone of claims 1 through 3, **characterized in that** the actuating element (218) is arranged at the outer end of the pivotable control element (214) and can be moved towards the control element (212) against the force of a spring element (244), whereby the brake elements (224, 225) can be moved away from the side walls (238, 238', 240) of the plate (236, 236').

5. Medical instrument of anyone of claims 1 through 4, **characterized in that** the brake elements (224, 225) are each formed as U-shaped straps, one end (228) of which is connected to the actuating element (218) and whose other end (230) can be positioned at a side wall (238, 238', 240) of the plate (236, 236').

6. Medical instrument of claim 5, **characterized in that** one end (228) of the U-shaped strap is mounted via a pivot bolt (223) at the actuating element (218), so that a movement of the actuating element (218) in direction to the plate (236, 236') causes the opposite end (230) of the U-shaped strap to move away from the respective side wall (238, 238', 240) of the plate (236, 236').

7. Medical instrument of anyone of claims 1 through 6, **characterized in that** the side walls (238. 238', 240) of the plate (236, 236') are roughened.

8. Medical instrument of anyone of claims 1 through 7, **characterized in that** the brake elements (224, 225) are provided with at least one brake body (226) in an area at which it are in contact with the side walls (238, 238', 240) of the plate (236, 236').

9. Medical instrument of anyone of claims 1 through 8, **characterized in that** at least one dimple (242) is provided in a side wall (238, 238', 240) of the plate (236, 236'), into which protrusions (232) of the brake elements (224, 225) can be inserted.

10. Medical instrument of claims 8 or 9, **characterized in that** a row on dimples (242) is provided, which are arranged along a circle whose radius origins from the pivot axis (214).

## Revendications

1. Instrument médical doté d'une tige (202), dont l'extrémité distale peut être pliée, d'un moyen de prise (204) agencé au niveau de l'extrémité proximale de la tige (202), d'un mécanisme de pliage (208) destiné à plier la tige (202), le mécanisme de pliage (208) présentant un organe de commande (212) pouvant pivoter autour d'un axe de pivot (214) et étant agencé au niveau d'un côté extérieur du moyen de prise (204), et dont le pivotement autour de l'axe de pivot (214) provoque un pliage de la tige (202), et d'un mécanisme de blocage destiné à bloquer l'organe de commande pivotable (212) dans différentes positions de pivotement, le mécanisme de blocage présentant un organe d'actionnement (218), dont l'actionnement libère le mécanisme de blocage, **caractérisé en ce qu'**une plaque (236, 236') est surélevée par rapport au côté extérieur du moyen de prise (204), des organes de frein (224, 225) se situant au niveau des parois latérales opposées (238, 238') de celle-ci, lesquels sont reliés à l'organe de commande pivotable (212) et lesquels sont précontraints dans la direction des parois latérales (238, 238', 240) de telle sorte que ceux-ci bloquent un pivotement de l'organe de commande pivotable (212), et **en ce que** l'organe d'actionnement (218) est relié aux organes de frein (224, 225) de telle sorte qu'un actionnement de l'organe d'actionnement (218) amène les organes de frein (224, 225) à quitter l'engagement bloqué avec la plaque (236).

2. Instrument médical selon la revendication 1, **caractérisé en ce que** l'organe de commande pivotable (212) peut être déplacé ensemble avec les organes de frein (224, 225) le long de la plaque (236, 236').

3. Instrument médical selon la revendication 1 ou 2, **caractérisé en ce que** l'organe de commande (218) est relié par l'intermédiaire d'un bras (216) à l'axe de pivot (214), et **en ce qu'**un bord extérieur de la plaque (236, 236') présente une courbure en forme de cercle, dont le rayon de courbure est le fruit de l'axe de pivot (214).

4. Instrument médical selon l'une des revendications 1 à 3, **caractérisé en ce que** l'organe d'actionnement (218) est agencé au niveau de l'extrémité extérieure de l'organe de commande pivotable (214) et peut être déplacé en s'opposant à la force d'un élément de ressort (244) vers l'organe de commande (212), grâce à quoi les organes de frein (224, 225) peuvent être écartés des parois latérales (238, 238', 240) de la plaque (236, 236').

5. Instrument médical selon l'une des revendications 1 à 4, **caractérisé en ce que** les organes de frein (224, 225) sont respectivement conçus sous la forme d'un étrier en forme de U, dont une première extrémité (228) est reliée à l'organe d'actionnement (218), et dont l'autre extrémité (230) peut être placée contre une paroi latérale (238, 238', 240) de la plaque (236, 236').

6. Instrument médical selon la revendication 5, **caractérisé en ce que** ladite première extrémité (228) de l'étrier en forme de U est positionnée sur un tourillon (223) au niveau de l'organe d'actionnement (218), de sorte qu'un déplacement de l'organe d'actionnement (218) dans la direction de la plaque (236, 236') entraîne un écartement par pivotement de l'autre extrémité (230) de l'étrier en forme de U par rapport à la paroi latérale respective (238, 238', 240) de la plaque (236, 236').

7. Instrument médical selon l'une des revendications 1 à 6, **caractérisé en ce que** les parois latérales (238, 238', 240) de la plaque (236, 236') sont rugueuses.

8. Instrument médical selon l'une des revendications 1 à 7, **caractérisé en ce que** les organes de frein (224, 225) sont dotés d'au moins un corps de frein (226) dans la zone où ceux-ci sont en contact avec les parois latérales (238, 238', 240) de la plaque (236, 236').

9. Instrument médical selon l'une des revendications 1 à 8, **caractérisé en ce qu'**au moins un évidement (242) est prévu dans une paroi latérale (238, 238', 240) de la plaque (236, 236'), dans lequel des parties en saillie (232) des organes de frein (224, 225) peuvent être engagées.

10. Instrument médical selon la revendication 8 ou 9, **caractérisé en ce qu'**une rangée d'évidements (242) est prévue, les évidements étant agencés le long d'un cercle, dont le rayon est le fruit de l'axe de pivot (214).
